# EUROPÄISCHE PATENTSCHRIFT

(11) **EP 3 852 699 B1**
(45) Veröffentlichungstag und Bekanntmachung des Hinweises auf die Patenterteilung: **04.09.2024**
(21) Anmeldenummer: 19808967.4
(22) Anmeldetag: 19.09.2019
(51) Int. Cl.: A61F 9/007

(54) **INSTRUMENT ZUM INTRAOKULAREN EINGRIFF**
INTRAOCULAR SURGICAL TOOL
INSTRUMENT D'OPÉRATION INTRAOCULAIRE

(30) Priorität: 19.09.2018 DE 102018215965
(43) Veröffentlichungstag der Anmeldung: 28.07.2021
(73) Patentinhaber: Geuder AG, 69126 Heidelberg (DE)
(72) Erfinder: STAPPLER, Theodor, 1815 Clarens (CH); EDER, Juergen, 69251 Gaiberg (DE); EL-AYARI, Hamadi, 60323 Frankfurt (DE)
(74) Vertreter: Ullrich & Naumann PartG mbB
(86) Internationale Anmeldenummer: PCT/DE2019/200111
(87) Internationale Veröffentlichungsnummer: WO 2020/057700

(56) Entgegenhaltungen:
- WO-A1-2005/094742
- US-A- 5 084 012
- US-A1- 2003 057 347

## Beschreibung

Die Erfindung, welche im Anspruch 1 definiert ist, betrifft ein Instrument zum intraokularen Eingriff, wobei es sich dabei im Konkreten um ein intraokulares Reinigungsinstrument handelt.

Instrumente zum intraokularen Eingriff sind hinlänglich aus der Praxis der Ophthalmologie bekannt. Dabei handelt es sich regelmäßig um zur Beleuchtung dienende Lichtleiter, Messer mit unterschiedlichster Ausprägung, Pinzetten, Vitrektoren zur Kernvitrektomie, etc. Die meisten dieser Instrumente verfügen über eine trokarfähige Sonde, d.h. über eine Sonde, die durch einen Trokar hindurch in das Auge einführbar ist. Ein Instrument nach der Präambel von Anspruch 1 ist aus US 5 084 012 A bekannt.

Bei dem hier im Rede stehenden Instrument handelt es sich um ein augenchirurgisches Instrument, mit einem Handgriff, einer sich an den Handgriff anschließenden, als Hohlkörper ausgeführten Sonde und einer in der Sonde angeordneten Seele.

Bei augenchirurgischen Eingriffen sind Verunreinigungen jedweder Art im Auge nicht zu vermeiden. Aus der Praxis ist es bereits bekannt, solche Verunreinigungen aus dem Auge - Vorderabschnitt oder Hinterabschnitt des Auges, abzusaugen. Aufgrund von Adhäsion ist dies nur bedingt möglich.

Der vorliegenden Erfindung liegt daher die Aufgabe zugrunde, ein wirksames Entfernen von "Verschmutzungen" durch ein intraokulares Reinigungsinstrument zu bewerkstelligen.

Dazu ist die Seele des zuvor erörterten Instruments endseitig, d.h. an ihrem distalen Ende, mit einem Wirkelement zum Beseitigen von Verunreinigungen im Auge ausgestattet. Letztendlich handelt es sich bei dem Wirkelement um ein Reinigungsgerät im weitesten Sinne, um beispielsweise Fremdkörper wie Silikontröpfchen aus dem Augeninneren von einer Membran oder dgl. Abzuwischen und zu entfernen.

Erfindungsgemäß dient das Wirkelement zur Reinigung, nämlich zur Beseitigung von Verunreinigungen im Auge. Das Instrument ist mit einer ganz besonderen Technik ausgestattet, nämlich dahingehend, dass entweder die Sonde zumindest teilweise aus dem Handgriff heraus und in diesen hinein gegenüber der fest mit dem Handgriff verbundenen Seele oder die Seele zumindest teilweise aus dem Handgriff heraus und in diesen hinein gegenüber der fest mit dem Handgriff verbundenen Sonde derart verschiebbar ist, dass sich die Seele mit dem Wirkelement komplett in der Sonde befindet (in einer Ruhestellung) oder um eine vorgegebene Länge aus der Sonde herausragt (in einer Arbeitsstellung), wobei im Handgriff eine Betätigungseinrichtung vorgesehen ist, durch die die Sonde oder Seele betätigbar ist.

Für die Erfindung ist wesentlich, dass die Seele mit dem endseitigen Wirkelement aus der Sonde heraus verlagerbar ist, um innerhalb des Auges an den jeweiligen Ort der Verunreinigung zu gelangen. Dabei sind zwei grundsätzliche Mechanismen denkbar, nämlich dahingehend, dass die Seele aus der Sonde zumindest teilweise herausgeschoben wird oder dass die Sonde, bei gegenüber dem Handgriff feststehender Seele, in den Handgriff zurück bzw. hineingeschoben wird. In beiden Fällen wird die Seele mit dem endseitigen Wirkelement exponiert und kann so an den jeweiligen Wirkort im Auge verbracht werden.

Das Wirkelement ist als Reinigungselement zu verstehen, wobei das Wirkelement im Konkreten als Pinsel, Mopp, Lappen, Schwamm, kleines Wischerblatt, als faseriges, flächiges, ballartiges Element, etc. ausgeführt sein kann. Auch kann es von Vorteil sein, wenn das Wirkelement zum Aufsaugen von Verunreinigungen geeignet ist, beispielsweise zum Aufsaugen von Silikonöltröpfchen, resultierend aus einer Operation/Behandlung am bzw. im Auge.

Entsprechend der Art des angestrebten Eingriffs kann die Seele im herausgeschobenen Zustand im Wesentlichen geradlinig ausgeführt sein, jedenfalls dann, wenn es möglich ist, die zu säubernde Stelle, beispielsweise an der Linse, geradlinig zu erreichen. Soll dagegen ein Bereich in der hinteren Augenkammer erreicht werden, kann es von Vorteil sein, wenn die Seele zum distalen Ende hin vorzugsweise um 45 Grad bis 180 Grad gebogen ist, derart, dass beim Herausschieben der Seele die Biegung sich unter elastischer Entspannung des Materials der Seele entfaltet und beim Hineinschieben der Seele diese eine elastische Vorspannung gegenüber der Innenwand der Sonde aufbaut.

Mit anderen Worten ist es mit dem erfindungsgemäßen Instrument möglich, zunächst bei eingeschobener Seele die Sonde durch einen Trokar und eine Inzision einzuführen und die Seele und das Wirkelement innerhalb des Auges zur Entfaltung zu bringen, wodurch ein besonders schonender Eingriff möglich ist.

Der Handgriff kann im Sinne eines Gehäuses ausgeführt sein, wobei er in vorteilhafter Weise als eine sich zum distalen Ende bzw. zur Sonde hin verjüngende Hülse ausgeführt sein kann. In dieser Hülse kann ein Betätigungselement einer Betätigungseinrichtung vorgesehen sein, durch dessen Verlagerung sich die Seele exponieren lässt. Das Betätigungselement kann als im Innern des Handgriffs laufende Innenhülse oder Bolzen ausgeführt sein, wobei es vorzugsweise über eine begrenzte Schiebestrecke hinweg läuft. Dadurch ist das Maß des Herausfahrens der Seele aus der Sonde definiert.

In weiter vorteilhafter Weise umfasst die Betätigungseinrichtung einen Feststellmechanismus zum Arretieren einer Relativlage zwischen Sonde und Seele. Dabei kann es sich im Konkreten um eine Klemmeinrichtung, eine Feststellschraube, etc. handeln.

In besonders vorteilhafter Weise ist im Handgriff ein Reservoir für eine Flüssigkeit ausgebildet, die durch die Sonde und gegebenenfalls durch die Seele zum Wirkelement hin ausbringbar ist. Bei dieser Flüssigkeit kann es sich beispielsweise um eine Kochsalzlösung handeln. Jedenfalls dient die Flüssigkeit gemeinsam mit dem Wirkelement zur Reinigung im weitesten Sinne.

Alternativ ist es denkbar, dass am proximalen Ende des Handgriffs ein Anschluss zum Anschließen bzw. Anstecken einer Spritze ausgebildet ist, deren Inhalt durch den Handgriff und durch die Sonde sowie gegebenenfalls durch die Seele zum Wirkelement hin ausbringbar ist. Durch Betätigen der Spritze wird die darin befindliche Flüssigkeit, im Konkreten durch Betätigung des Kolbens der Spritze, durch den Handgriff bzw. das Gehäuse hindurch und durch die Sonde ausgebracht.

Das erfindungsgemäße Instrument kann am proximalen Ende des Handgrifft einen besonderen Anschluss zum Verbinden mit einem Vitrektomiegerät mit Infusionsund Aspirationsanschlüssen mittels Schlauch haben. Durch eine solche Ausgestaltung und die Vorkehrung eines üblichen oder kodierten Anschlusses, lässt sich das Instrument an ein Vitrektomiegerät anschließen, so dass es mit einer Spül/Reinigungsflüssigkeit gespeist werden kann. Auch ist es denkbar, über das Vitrektomiegerät abzusaugen.

Der Handgriff und/oder die Sonde kann aus Kunststoff oder Metall, insbesondere aus Edelstahl, hergestellt sein. Insbesondere bei Einmalgeräten bietet sich die preiswerte Herstellung aus Kunststoff an.

Die Seele kann ebenfalls aus Kunststoff hergestellt sein, wobei es sich bei einer im herausgefahrenen Zustand gebogenen Seele um eine Seele aus Kunststoff mit Formgedächtnis handeln kann. Ebenso ist es denkbar, eine Metalllegierung mit Formgedächtnis zu verwenden, beispielsweise eine Nickel-Titan-Formgedächtnislegierung, wie sie auf unterschiedlichsten technischen Gebieten, für sich gesehen, bekannt ist.

Bereits zuvor ist ausgeführt worden, dass die Maße der Sonde derart auszulegen sind, dass die Sonde durch einen handelsüblichen Trokar in das Auge einführbar ist. Dabei kann die Sonde einen Durchmesser von 0,4 bis 0,8 mm haben, vorzugsweise im Bereich von etwa 0,6 mm.

Die Seele kann einen Durchmesser von 0,4 bis 0,5 mm haben, vorzugsweise 0,45 mm. Das Reinigungsmaterial, welches am distalen Ende der Seele angebracht ist, könnte im gebündelten bzw. zusammengepressten Zustand ein Durchmesser von 0,35 mm haben, wobei sich das Material vorzugsweise beim Herausschieben des distalen Bereichs der Seele aus der Sonde entfaltet, beispielsweise durch Aufspleißen von drei oder vier lappen- bzw. faserartigen Wischelementen.

Das erfindungsgemäße Instrument kann durch einen Pars-Plana gesetzten Trokar in das Auge eingeführt werden, nämlich durch eine Minimalinzision, zumal sich das Wirk- bzw. Reinigungselement im eingefahrenen Zustand in der Sonde befindet. Es ist dort regelrecht zusammengequetscht. Entsprechend der gewünschten Richtung in das Auge hinein, ausgehend vom Pars-Plana-Zugang, lässt sich die Seele mit dem endseitigen Wirkelement ausfahren, wodurch das Wirkelement eine Vergrößerung erfährt. Reinigungsmittel können dann, durch einen inneren Kanal, hinzugegeben werden, entweder aus einem Reservoir im Handgriff heraus, durch eine angeschlossene Spritze oder durch aktive Infusion und Aspiration, nämlich über einen Anschluss an ein Vitrektomiegerät mit Infusions- und Aspirationsanschlüssen. Das Ein- und Ausfahren des Wirkelements und das mehr oder weniger starke Biegen der Seele mit dem endseitigen Wirkelement kann durch unterschiedlichste Schiebemechanismen am bzw. im Handgriff erfolgen.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Seitenansicht ein Ausführungsbeispiel eines erfindungsgemäßen Instruments mit nur geringfügig herausgeschobener, geradliniger Seele und endseitigem Wirkelement,
- Fig. 2: in schematischer Ansicht, vergrößert, die Sonde mit geringfügig herausgeschobener Seele und endseitigem Wirkelement,
- Fig. 3: in einer schematischen Ansicht das Instrument aus Figur 1 mit weit herausgeschobener und um 180 Grad gebogener Seele und endseitigem Wirkelement,
- Fig. 4: in einer schematischen Ansicht, vergrößert, die Sonde mit weit herausgeschobener Seele und endseitigem Wirkelement.
- Fig. 5: in einer schematischen Vorderansicht die endseitige Seele mit entfaltetem Wirkelement, gegenüber dem eingeschobenen Zustand mit vergrößerter Oberfläche und
- Fig. 6: in einer schematischen Ansicht zwei Eingriffssituationen mit dem erfindungsgemäßen Instrument am/im menschlichen Auge.

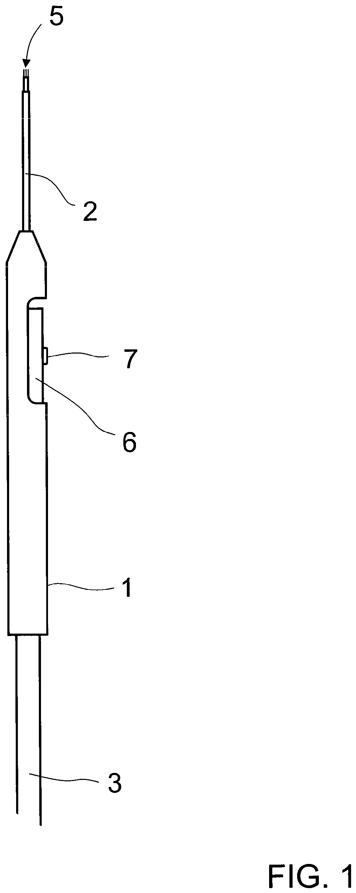

Figur 1 zeigt ein Ausführungsbeispiel eines erfindungsgemäßen Instruments zum intraokularen Eingriff. Das Instrument umfasst einen Handgriff 1 und eine sich an den Handgriff 1 anschließende Sonde 2.

Am proximalen Ende des Handgriffs 1 ist ein Spül- und Sauganschluss 3 vorgesehen.

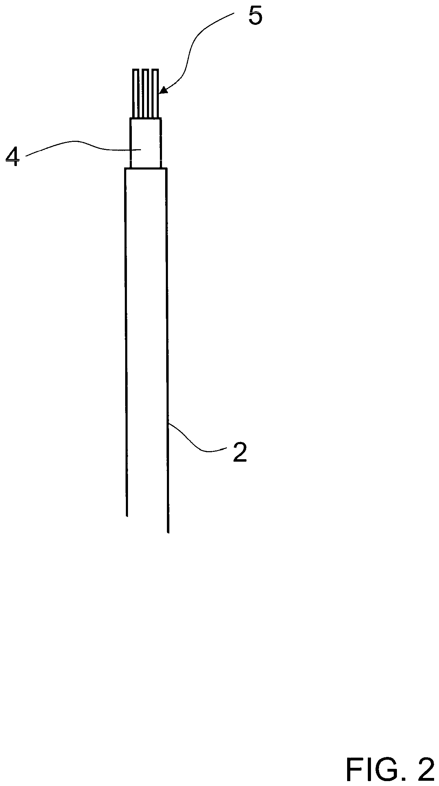

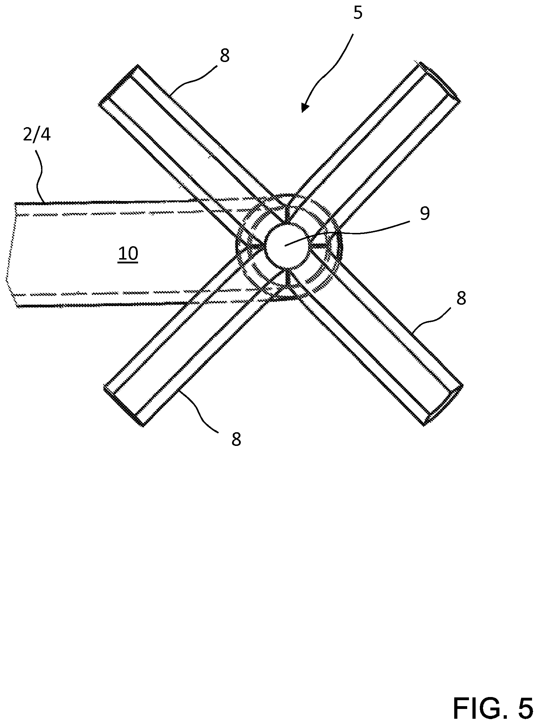

Figur 2 zeigt den Gegenstand aus Figur 1 im Bereich des distalen Endes, nämlich im Konkreten die Sonde 2 und die wenig bzw. gering herausgefahrene Seele 4, die sich geradlinig aus der Sonde 2 heraus erstreckt. Endseitig an der Seele 4 ist ein Wirkelement 5 vorgesehen wie es in Figur 5 im Detail als eine von vielen Möglichkeiten gezeigt wird. Es besteht aus einem Reinigungsmaterial mit vergrößerter Oberfläche.

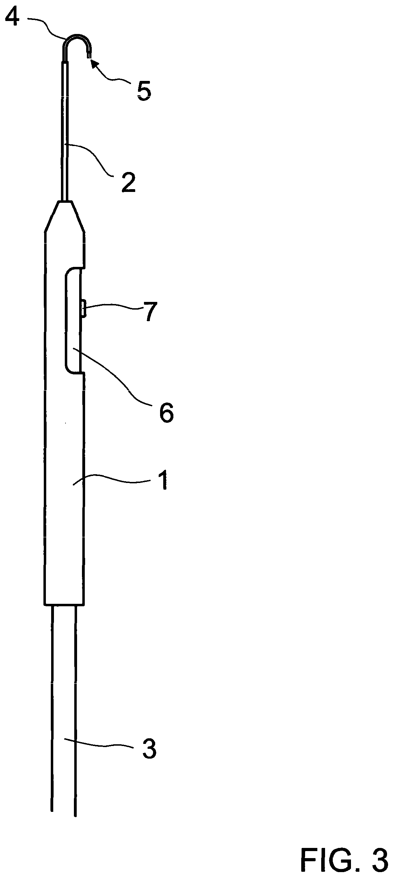

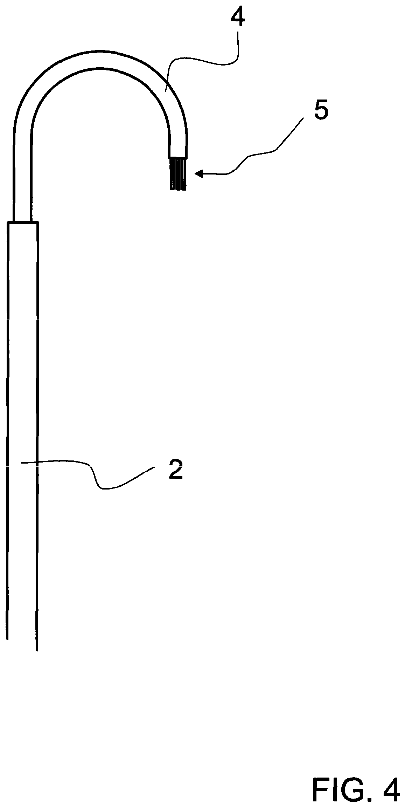

Figur 3 zeigt den Gegenstand aus Figur 1, wobei die Seele 4 weit herausgeschoben ist. Da es sich bei dem Material der Seele 4 um ein gebogenes Material mit Formgedächtnis handelt, biegt sich die Seele 4 beim Herausschieben aus der Sonde 2 entsprechend dem Formgedächtnis, gemäß Figur 4 um etwa 180 Grad. Endseitig an der Seele 4 ist das Wirkelement 5 angeordnet.

An dieser Stelle sei angemerkt, dass die Seele 4 auch gerade soweit herausgeschoben werden kann, dass das Wirkelement 5 etwa orthogonal von der Längsrichtung der Sonde 2 abragt.

Die Figuren 1 und 3 lassen des Weiteren erkennen, dass das Gehäuse eine Betätigungseinrichtung mit Betätigungselement 6 umfasst. Zum Arretieren der Position der Seele 4 gegenüber der Sonde 2 ist ein Feststellorgan 7 eines Feststellmechanismus vorgesehen.

Figur 5 zeigt die endseitige Situation am distalen Ende der Sonde 2 bzw. Seele 4, wonach endseitig an der Seele 4 das Wirkelement 5 angeordnet ist. Bei dem in Figur 5 gezeigten Beispiel entfaltet sich das Wirkelement 5 moppartig und umfasst vier Reinigungslappen 8, die sich beim Herausschieben der Seele 4 aus der Sonde 2 entfalten. Aufgrund ihres Materials sind sie geeignet, Verunreinigungen aufzunehmen. Mittig im Wirkelement 5 kann eine zum Absaugen dienende Öffnung 9 vorgesehen sein, die in einen inneren Kanal 10 mündet.

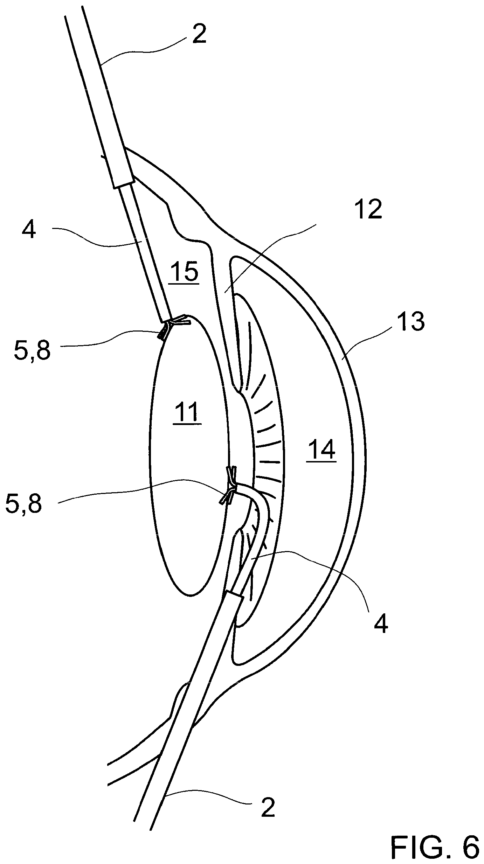

Figur 6 zeigt schließlich zwei verschiedene Situationen im Rahmen eines intraokularen Eingriffs, wobei das erfindungsgemäße Instrument lediglich angedeutet ist. Es ist die Sonde 2 und die Seele 4 erkennbar, wobei zwei unterschiedliche Instrumente oder Arbeitszustände mit geradliniger Seele 4 und mit gebogener Seele 4 abgebildet sind. In beiden Fällen ist am distalen Ende der Seele 4 das Wirkelement 5 entsprechend den voranstehenden Ausführungen gezeigt. In beiden Fällen dient das erfindungsgemäße Instrument zur Reinigung an der Linsenoberfläche.

Einer vollständigen Darstellung halber ist in der schematischen Ansicht gemäß Figur 6 die Linse 11, die Iris 12, die Kornea 13 sowie die vordere Augenkammer 14 und die hintere Augenkammer 15 angedeutet.

Hinsichtlich weiterer vorteilhafter Ausgestaltungen des erfindungsgemäßen Instruments wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel des erfindungsgemäßen Instruments lediglich zur Erörterung der beanspruchten Lehre dient, diese jedoch nicht auf das Ausführungsbeispiel einschränkt.

### Bezugszeichenliste

- 1: Handgriff
- 2: Sonde
- 3: Spül- und Sauganschluss
- 4: Seele
- 5: Wirkelement
- 6: Betätigungselement
- 7: Feststellorgan
- 8: Reinigungslappen
- 9: Öffnung
- 10: innerer Kanal
- 11: Linse
- 12: Iris
- 13: Kornea
- 14: vordere Augenkammer
- 15: hintere Augenkammer

## Patentansprüche

1. Instrument zum intraokularen Eingriff, mit einem Handgriff (1), einer sich an den Handgriff (1) anschließenden, als Hohlkörper ausgeführten Sonde (2) und einer in der Sonde (2) angeordneten Seele (4) mit einem endseitigen Wirkelement (5) zum Beseitigen von Verunreinigungen im Auge, wobei entweder die Sonde (2) zumindest teilweise aus dem Handgriff (1) heraus und in diesen hinein gegenüber der fest mit dem Handgriff (1) verbundenen Seele (4) oder die Seele (4) zumindest teilweise aus dem Handgriff (1) heraus und in diesen hinein gegenüber der fest mit dem Handgriff (1) verbundenen Sonde (2) derart verschiebbar ist, dass sich die Seele (4) mit dem Wirkelement (5) komplett in der Sonde (2) befindet oder um eine vorgegebene Länge aus der Sonde (2) heraus ragt, wobei im Handgriff (1) eine Betätigungseinrichtung vorgesehen ist, durch die die Sonde (2) oder Seele (4) betätigbar ist, **dadurch gekennzeichnet, dass** das Wirkelement (5) zur Reinigung dient und als Mopp, Lappen, , Wischerblatt, oder ballartig ausgebildet ist.

2. Instrument nach Anspruch 1, **dadurch gekennzeichnet, dass** die Seele (4) im herausgeschobenen Zustand im Wesentlichen geradlinig ausgeführt ist.

3. Instrument nach Anspruch 1, **dadurch gekennzeichnet, dass** die Seele (4) zum distalen Ende hin vorzugsweise um 45° bis 180° gebogen ist, derart, dass beim Herausschieben der Seele (4) die Biegung sich unter elastischer Entspannung entfaltet und beim Hineinschieben der Seele (4) diese eine elastische Vorspannung gegenüber der Innenwand der Sonde (2) aufbaut.

4. Instrument nach einem der Ansprüche 1 bis 3, **dadurch gekennzeichnet, dass** der Handgriff (1) als sich vorzugsweise am distalen Ende zur Sonde (2) hin verjüngende Hülse ausgeführt ist, in der ein Betätigungselement (6) der Betätigungseinrichtung als Innenhülse, Bolzen oder dgl. vorzugsweise über eine begrenzte Schiebestrecke hinweg, läuft.

5. Instrument nach Anspruch 4, **dadurch gekennzeichnet, dass** die Betätigungseinrichtung einen Feststellmechanismus zum Arretieren einer Relativlage zwischen Sonde (2) und Seele (4) umfasst.

6. Instrument nach einem der Ansprüche 1 bis 5, **dadurch gekennzeichnet, dass** im Handgriff (1) ein Reservoir für eine Flüssigkeit ausgebildet ist, die durch die Sonde (2) und ggf. durch die Seele (4) zum Wirkelement (5) hin ausbringbar ist.

7. Instrument nach einem der Ansprüche 1 bis 5, **dadurch gekennzeichnet, dass** am proximalen Ende des Handgriffs (1) ein Anschluss zum Anschließen bzw. Anstecken einer Spritze ausgebildet ist, deren Inhalt durch den Handgriff (1) und durch die Sonde (2) sowie ggf. durch die Seele (4) zum Wirkelement (5) hin ausbringbar ist.

8. Instrument nach einem der Ansprüche 1 bis 5, **dadurch gekennzeichnet, dass** am proximalen Ende des Handgriffs (1) ein Anschluss zur Verbindung mit einem Vitrektomie gerät mit Infusions- und Aspirationsanschlüssen mittels Schlauch ausgebildet ist.

9. Instrument nach einem der Ansprüche 1 bis 8, **dadurch gekennzeichnet, dass** der Handgriff (1) und/oder die Sonde (2) aus Kunststoff oder Metall, insbesondere aus Edelstahl, hergestellt ist.

10. Instrument nach einem der Ansprüche 1 bis 9, **dadurch gekennzeichnet, dass** die Seele (4) aus Kunststoff mit einem Formgedächtnis oder einer Metalllegierung mit Formgedächtnis, beispielsweise aus einer Nickel-Titan-Formgedächtnislegierung, hergestellt ist.

11. Instrument nach einem der Ansprüche 1 bis 10, **dadurch gekennzeichnet, dass** die Maße der Sonde (2) derart ausgelegt sind, dass die Sonde (2) durch einen handelsüblichen Trokar in das Auge einführbar ist, beispielsweise einen Durchmesser von 0,4 bis 0,8 mm, vorzugsweise 0,6 mm, hat.

## Claims

1. Instrument for intraocular intervention, having a handle (1), a probe (2) which adjoins the handle (1) and which is in the form of a hollow member and a core (4) which is arranged in the probe (2) and which has an end-side active element (5) for eliminating contaminations in the eye, wherein either the probe (2) can be at least partially displaced out of and into the handle (1) with respect to the core (4) which is securely connected to the handle (1) or the core (4) can be at least partially displaced out of and into the handle (1) with respect to the probe (2) which is securely connected to the handle (1) in such a manner that the core (4) with the active element (5) is located completely in the probe (2) or protrudes by a predetermined length out of the probe (2), wherein in the handle (1) there is provided an activation device by means of which the probe (2) or core (4) can be activated,
**characterised in that** the active element (5) is used for cleaning and is in the form of a mop, cloth, wiper blade or is constructed in a ball-like manner.

2. Instrument according to claim 1, **characterised in that** the core (4) in the pushed-out state is configured in a substantially linear manner.

3. Instrument according to claim 1, **characterised in that** the core (4) is bent in the direction towards the distal end, preferably through from 45° to 180°, in such a manner that, when the core (4) is pushed out, the bending is carried out under resilient relaxation and, when the core (4) is pushed in, it produces a resilient pretensioning with respect to the inner wall of the probe (2).

4. Instrument according to any one of claims 1 to 3, **characterised in that** the handle (1) is in the form of a sleeve which preferably tapers at the distal end in the direction towards the probe (2) and in which an activation element (6) of the activation device in the form of an inner sleeve, pin or the like preferably runs over a limited sliding path.

5. Instrument according to claim 4, **characterised in that** the activation device comprises a fixing mechanism for locking a relative position between the probe (2) and core (4) .

6. Instrument according to any one of claims 1 to 5, **characterised in that** in the handle (1) there is formed a tank for a fluid which can be discharged through the probe (2) and where applicable through the core (4) to the active element (5).

7. Instrument according to any one of claims 1 to 5, **characterised in that** there is constructed at the proximal end of the handle (1) a connection for connecting or fitting a syringe whose content can be discharged through the handle (1) and through the probe (2) and where applicable through the core (4) in the direction towards the active element (5).

8. Instrument according to any one of claims 1 to 5, **characterised in that** at the proximal end of the handle (1) a connection for connection to a vitrectomy device having infusion and aspiration connections is formed by means of a hose.

9. Instrument according to any one of claims 1 to 8, **characterised in that** the handle (1) and/or the probe (2) is produced from plastics material or metal, in particular from high-grade steel.

10. Instrument according to any one of claims 1 to 9, **characterised in that** the core (4) is produced from plastics material with shape memory or a metal alloy with shape memory, for example, from a nickel/titanium shape-memory alloy.

11. Instrument according to any one of claims 1 to 10, **characterised in that** the dimensions of the probe (2) are configured in such a manner that the probe (2) can be introduced into the eye by means of a commercially available trocar, for example, has a diameter of from 0.4 to 0.8 mm, preferably 0.6 mm.

## Revendications

1. Instrument pour une intervention intra-oculaire, avec une poignée (1), une sonde (2), conçue comme un corps creux, se raccordant à la poignée (1) et une âme (4) disposée dans la sonde (2), avec un élément actif côté extrémité (5) pour l'élimination des impuretés dans l'œil, dans lequel, soit la sonde (2) peut être déplacée au moins partiellement hors de la poignée (1) et rentrée dans celle-ci par rapport à l'âme (4) reliée fermement avec la poignée (1), soit l'âme (4) peut être déplacée au moins partiellement hors de la poignée (1) et rentrée dans celle-ci par rapport à la sonde (2) reliée fermement avec la poignée (1), de sorte que l'âme (4) avec l'élément actif (5) se trouve entièrement dans la sonde (2) ou dépasse d'une longueur prédéterminée hors de la sonde (2), dans lequel, dans la poignée (1), est prévu un dispositif d'actionnement qui permet d'actionner la sonde (2) ou l'âme (4),
**caractérisé en ce que** l'élément actif (5) permet le nettoyage et présente la forme d'une serpillière, d'un chiffon, d'un balai d'essuie-glace ou la forme d'une balle.

2. Instrument selon la revendication 1, **caractérisé en ce que** l'âme (4) présente globalement, dans l'état déployé, la forme d'une ligne droite.

3. Instrument selon la revendication 1, **caractérisé en ce que** l'âme (4) est pliée en direction de l'extrémité distale de préférence de 45° à 180°, de sorte que, lors de la poussée de l'âme (4) vers l'extérieur, le pliage se déplie sous l'action d'une détente élastique et, lors de la rétraction de l'âme (4), celle-ci génère une précontrainte élastique par rapport à la paroi interne de la sonde (2).

4. Instrument selon l'une des revendications 1 à 3, **caractérisé en ce que** la poignée (1) est conçue comme un manchon se rétrécissant, de préférence au niveau de l'extrémité distale, en direction de la sonde (2), dans lequel s'étend un élément d'actionnement (6) du dispositif d'actionnement sous la forme d'un manchon interne, d'une tige ou autre, de préférence au-delà d'une trajectoire de poussée limitée.

5. Instrument selon la revendication 4, **caractérisé en ce que** le dispositif d'actionnement comprend un mécanisme de blocage pour la fixation d'une position relative entre la sonde (2) et l'âme (4).

6. Instrument selon l'une des revendications 1 à 5, **caractérisé en ce que**, dans la poignée (1), est réalisé un réservoir pour un liquide, qui peut être amené, à travers la sonde (2) et, le cas échéant, à travers l'âme (4), vers l'élément actif (5).

7. Instrument selon l'une des revendications 1 à 5, **caractérisé en ce que**, au niveau de l'extrémité proximale de la poignée (1), est prévu un raccord pour le raccordement ou l'enfichage d'une seringue dont le contenu peut être amené à travers la poignée (1) et à travers la sonde (2) ainsi que, le cas échéant, à travers l'âme (4), vers l'élément actif (5).

8. Instrument selon l'une des revendications 1 à 5, **caractérisé en ce que**, au niveau de l'extrémité proximale de la poignée (1), est prévu un raccord pour la liaison avec un appareil de vitrectomie avec des raccords d'injection et d'aspiration au moyen d'un tuyau.

9. Instrument selon l'une des revendications 1 à 8, **caractérisé en ce que** la poignée (1) et/ou la sonde (2) sont constituées de matière plastique ou de métal, plus particulièrement d'acier inoxydable.

10. Instrument selon l'une des revendications 1 à 9, **caractérisé en ce que** l'âme (4) est constituée d'une matière plastique à mémoire de forme ou d'un alliage métallique à mémoire de forme, par exemple d'un alliage nickel-titane à mémoire de forme.

11. Instrument selon l'une des revendications 1 à 10, **caractérisé en ce que** les dimensions de la sonde (2) sont telles que la sonde (2) peut être introduite dans l'œil à l'aide d'un trocart disponible dans le commerce, par exemple un diamètre de 0,4 à 0,8 mm, de préférence de 0,6 mm.
